# FASCICULE DE BREVET EUROPEEN

(11) **EP 1 557 155 B1**
(45) Date de publication et mention de la délivrance du brevet: **02.05.2007**
(21) Numéro de dépôt: 04293076.8
(22) Date de dépôt: 22.12.2004
(51) Int. Cl.: A61K 8/46, A61K 8/44, A61K 8/81, A61K 8/84, A61Q 5/02, A61Q 5/12, A61K 8/20, A61K 8/19

(54) **Composition cosmétique détergente comprenant des tensioactifs anioniques et amphotères, un polymère cationique fortement chargé et un sel hydrosoluble**
Reinigende kosmetische Zusammensetzung enthaltende anionische und amphoterische Tenside, eine hochgeladene kationische Polymere und ein wasserlösliches Salz
Cleansing cosmetic composition containing anionic and amphoteric surfactants, a highly charged cationic polymer and a water-soluble salt

(30) Priorité: 05.01.2004 FR 0400027
(43) Date de publication de la demande: 27.07.2005
(73) Titulaire: L'ORÉAL, 75008 Paris (FR)
(72) Inventeur: Simonet, Frédéric, 77131 Touquin (FR)
(74) Mandataire: Dossmann, Gérard

(56) Documents cités:
- EP-A- 1 025 839
- WO-A-00/45781
- WO-A-03/032935
- WO-A-03/101410
- FR-A- 2 825 269
- US-A- 5 747 436
- US-A- 5 977 037

## Description

La présente invention concerne une composition cosmétique détergente comprenant une base lavante particulière, un polymère cationique à densité de charge élevée et un sel hydrosoluble, l'utilisation de ladite composition pour le lavage et le conditionnement des matières kératiniques, et un procédé de traitement cosmétique des matières kératiniques mettant en oeuvre cette composition.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés, notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Dans ce but, les polymères cationiques, en particulier ceux fortement chargés, peuvent être utilisés du fait de leur pouvoir conditionneur particulièrement élevé, notamment sur les cheveux sensibilisés.

Cependant, ils présentent l'inconvénient de former un complexe avec des composés anioniques et de précipiter, par exemple, lorsqu'on les introduit dans une base lavante comprenant des tensioactifs anioniques et amphotères.

Il est donc difficile de conserver un caractère homogène et de bonnes qualités d'usage telles que mousse abondante, viscosité acceptable et ajustable par ajout d'un tiers composé dans une composition comprenant de tels polymères cationiques et une base

Des exemples de telles compositions sont décrits dans les documents EP 1 025 839 et WO 00/45781. Le document EP 1 025 839 divulgue une composition cosmétique, détergente et conditionnante, comprenant une base lavante à base de tensioactifs anionique et amphotère, une huile de polyoléfine de poids moléculaire inférieur à 450, un polymère cationique et un sel hydrosoluble et/ou un alcool hydrosoluble mono- ou polyhydroxylé, le rapport en poids tensioactif anionique/tensioactif amphotère étant inférieur ou égal à 3. Le document WO 00/45781 décrit quant à lui une composition cosmétique, détergente et conditionnante, comprenant une base lavante à base de tensioactifs anionique et amphotère en un rapport pondéral tensioactif anionique/tensioactif amphotère inférieur ou égal à 3, au moins 1 % d'ester d'acide carboxylique particulier insoluble dans l'eau, la composition étant exempte de tensioactif cationique.

La demanderesse a découvert de manière surprenante que l'on pouvait obtenir des compositions détergentes homogènes comprenant une quantité non négligeable de polymères cationiques fortement chargés en utilisant au moins un tensioactif anionique et au moins un tensioactif amphotère particulier en un rapport pondéral tensioactif(s) anionique(s)/tensioactif(s) amphotère(s) inférieur ou égal à 1, une quantité minimale d'au moins un sel hydrosoluble particulier, et une quantité totale de tensioactifs inférieure ou égale à 18% en poids par rapport au poids total de la composition. Cette composition permet de surmonter les inconvénients présentés ci-dessus.

En outre, ces compositions présentent des viscosités acceptables (viscosité > 2 Pa.s à 25 °C à un taux de cisaillement inférieur ou égal à 1 s⁻¹, mesurée avec un rhéomètre cône-plan du type ThermoRheo RS1) qui peuvent être ajustées par simple ajout de sel ou de fluidifiant selon les exigences industrielles, tout en restant limpides.

L'invention a donc pour objet une composition cosmétique détergente comprenant, dans un milieu aqueux cosmétiquement acceptable, au moins un tensioactif anionique et au moins un tensioactif amphotère tel que décrit ci-dessous en un rapport pondéral tensioactif(s) anionique(s)/tensioactif(s) amphotère(s) inférieur ou égal à 1, au moins un polymère cationique présentant une densité de charge cationique supérieure à 5 meq/g, et au moins 1% en poids d'un sel hydrosoluble minéral ou organique, l'anion de ce dernier comprenant, s'il est organique, de 1 à 7 atomes de carbone, et comprenant une quantité totale de tensioactifs inférieure ou égale à 18 % en poids par rapport au poids total de la composition.

Un autre objet de l'invention est l'utilisation de la composition pour le lavage et/ou le conditionnement des matière kératiniques telles que les cheveux.

L'invention a encore pour objet un procédé de traitement cosmétique des matières kératiniques mettant en oeuvre la composition selon l'invention.

D'autres objets, caractéristiques, aspects et avantages de l'invention apparaîtront encore plus clairement à la lecture de la description et des divers exemples qui suivent.

Selon l'invention, la composition cosmétique détergente comprend dans un milieu aqueux cosmétiquement acceptable,
- au moins un tensioactif anionique et au moins un tensioactif amphotère choisi parmi les alkyl(C₈-C₂₄)amidoalkyl(C₃-C₈)-bétaïnes, les sulfobétaines, les alkyl(C₈-C₂₄)amidoalkyl(C₆-C₈)sulfobétaïnes, les alkyl(C₈-C₂₄)amphomonoacétates, les alkyl(C₈-C₂₄)amphodiacétates, les alkyl(C₈-C₂₄)amphomonopropionates, les alkyl(C₈-C₂₄)amphodipropionates et les phosphobétaïnes, en un rapport pondéral tensioactif(s) anionique(s)/tensioactif(s) amphotère(s) inférieur ou égal à 1, de préférence compris entre 0,1 et 1, et mieux encore entre 0,2 et 1,
- au moins un polymère cationique présentant une densité de charge cationique supérieure à 5 méq/g,
- au moins 1 % en poids, par rapport au poids total de la composition, d'au moins un sel hydrosoluble minéral ou organique, l'anion de ce dernier comprenant, s'il est organique, de 1 à 7 atomes de carbone,
la quantité totale de tensioactifs dans la composition étant inférieure ou égale à 18 % en poids par rapport au poids total de la composition.

Par "composition détergente", on entend au sens de la présente invention une composition comportant au moins 4% en poids de tensioactifs anioniques et amphotères, et éventuellement de tensioactifs non ioniques, par rapport au poids total de la composition.

Par "milieu cosmétiquement acceptable", on entend un milieu compatible avec toutes les matières kétatiniques telles que la peau, les cheveux, les ongles, les cils, les sourcils, les lèvres et toute autre zone du corps et du visage, mais aussi d'odeur, d'aspect et de toucher agréables.

Avantageusement, le polymère cationique est soluble dans le milieu.

Par "sel hydrosoluble", on entend au sens de la présente invention un sel présentant une solubilité dans l'eau à 25°C supérieure à 1%, c'est-à-dire formant à cette concentration un milieu macroscopiquement homogène, transparent et isotrope.

De préférence, la composition est transparente.

Par "composition transparente", on entend une composition présentant une turbidité inférieure ou égale à 300 NTU, les NTU étant les unités néphélométriques de mesure de la turbidité.

La turbidité peut être mesurée, par exemple, avec un turbidimètre Model 2100P commercialisé par la société HACH Company, les tubes utilisés pour la mesure étant référencés AR397A cat 24347-06.

L'étalonnage est effectué avec de la formazine et les mesures sont effectuées à température ambiante (20 à 25 °C). Les compositions de l'invention ont de préférence une turbidité allant de 0,05 à 100 NTU.

A titre de tensioactifs anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle.

On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 10 à 24 atomes de carbone. Parmi les tensioactifs anioniques encore utilisables, on peut également citer les lactylates d'acyle dont le groupe acyle comporte de 8 à 24 atomes de carbone.

En outre, on peut encore citer les acides d'alkyl-D-galactoside uroniques et leurs sels ainsi que les acides alkyl(C₁₀-C₂₄)éthercarboxyliques polyoxyalkylénés, les acides alkyl(C₁₀-C₂₄)aryl(C₆-C₂₄)éther-carboxyliques polyoxyalkylénés, les acides alkyl(C₁₀-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sulfates d'alkyle, les alkyléther-sulfates comme le lauryléthersulfate de sodium, de préférence à 2 ou 3 moles d'oxyde d'éthylène, les alkyléthercarboxylates, les groupes alkyle comportant généralement de 10 à 24 atomes de carbone, et de préférence de 10 à 16 atomes de carbone, sous la forme particulière de sels de sodium, de magnésium ou d'ammonium.

Les agents tensioactifs amphotères convenant dans la présente invention sont choisis parmi les alkyl(C₁₀-C₂₄)amidoalkyl(C₃-C₈)-bétaïnes, les sulfobétaïnes, les alkyl(C₁₀-C₂₄)amidoalkyl(C₆-C₈)sulfobétaïnes, les alkyl(C₁₀-C₂₄)amphomonoacétates, les alkyl(C₁₀-C₂₄)amphodiacétates, les alkyl(C₁₀-C₂₄) amphomonopropionates, les alkyl(C₁₀-C₂₄) amphodipropionates, les phosphobétaïnes, et leurs mélanges.

Parmi les tensioactifs amphotères utilisables dans la composition selon l'invention, on peut notamment citer les produits commercialisés sous la dénomination MIRANOL^{®}, tels que décrits dans les brevets US-2 528 378 et US-2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N⁺(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   R_{a'}-CONHCH₂CH₂-N(B)(E) (2)

   dans laquelle :

B représente -CH₂CH₂OX',
E représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R_{a'} représente un groupe alkyle d'un acide R_{a'}-COOH présent dans l'huile de coprah ou dans l'huile de lin hydrolysée, un groupe alkyle, notamment en C₁₇ ou sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL^{®} C2M concentré par la société RHODIA.

De préférence, les tensioactifs amphotères sont choisis parmi les alkyl(C₁₀-C₂₄)amidoalkyl(C₃-C₈)-bétaïnes et les alkyl(C₁₀-C₂₄)-amphodiacétates.

De préférence, le(s) tensioactif(s) anionique(s) est ou sont présent(s) en une quantité allant de 0,4 à 9% en poids, mieux encore de 4 à 8% en poids par rapport au poids total de la composition.

De préférence, le(s) tensioactif(s) amphotère(s) est ou sont présent(s) en une quantité allant de 2 à 16% en poids, mieux encore de 4 à 12% en poids par rapport au poids total de la composition.

La composition selon l'invention peut comprendre en outre d'autres tensioactifs que ceux définis ci-dessus, tels que des tensioactifs non ioniques ou cationiques bien connus dans la technique.

La composition selon l'invention comprend une quantité totale de tensioactifs englobant les tensioactifs anioniques, amphotères, non ioniques et cationiques, inférieure ou égale à 18 % en poids, de préférence comprise entre 4 et 18 % en poids, et encore plus préférentiellement entre 4 et 15 % en poids par rapport au poids total de la composition cosmétique.

La composition cosmétique détergente selon l'invention comprend un ou plusieurs polymères cationiques dont la densité de charge cationique est strictement supérieure à 5 milliéquivalents par gramme, de préférence comprise entre 5 et 20 méq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl.

Les polymères cationiques ayant une densité de charge cationique supérieure à 5 méq/g, utilisables conformément à la présente invention, peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière générale, au sens de la présente invention, l'expression "polymère cationique" désigne tout polymère contenant des groupes cationiques et/ou des groupes ionisables en groupes cationiques.

Les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les copolymères vinylpyrrolidone/(méth)acrylate de dialkylaminoalkyle quaternisés ou non,
(2) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bishaloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bishaloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alkylés ou s'ils comportent une ou plusieurs fonctions amines tertiaires, quaternisés. De tels polymères sont notamment décrits dans les brevets français 2 252 840 et 2 368 508 ;
(4) les dérivés de polyaminoamides résultant de la condensation de polyalkylènes-polyamines avec des acides polycarboxyliques suivie d'une alkylation par des agents bifonctionnels. On peut citer, par exemple, les polymères acide adipique/diakylaminohydroxyalkyl-dialkylène-triamine dans lesquels le groupe alkyle comporte de 1 à 4 atomes de carbone et désigne de préférence méthyle, éthyle, propyle. De tels polymères sont notamment décrits dans le brevet français 1 583 363.
(5) les polymères obtenus par réaction d'une polyalkylène-polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(6) les cyclopolymères d'alkyldiallylamine ou de dialkyldiallylammonium tels que les homopolymères ou copolymères comportant des motifs répondant aux formules (VI) ou (VI') : 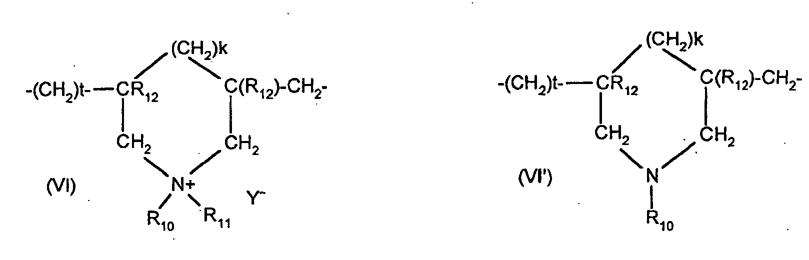 formules dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur, ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   On peut citer, par exemple, l'homopolymère de chlorure de diallyldiméthylammonium commercialisé sous la dénomination "MERQUAT^{®} 100" par la société ONDEO-NALCO et les copolymères de chlorure de diallyldiméthylammonium et d'acrylamide.
(7) le polycondensat de diammonium quaternaire contenant des motifs récurrents répondant à la formule : 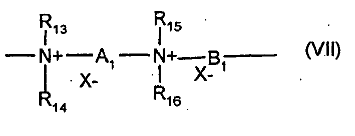 formule (VII) dans laquelle :
   R₁₃, R₁₄, R₁₅ et R₁₆, identiques ou différents, représentent des groupes aliphatiques, alicycliques, ou aryle aliphatiques contenant de 1 à 20 atomes de carbone ou des groupes hydroxyalkyle aliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique ;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement -(CH₂)ₙ-CO-D-OC-(CH₂)ₙ- dans lequel D désigne :
      a) un reste de glycol de formule : -O-Z-O-, où Z désigne un groupe hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un groupe hydrocarboné linéaire ou ramifié, ou bien le groupe bivalent

         -CH₂-CH₂-S-S-CH₂-CH₂- ;
      d) un groupement uréylène de formule : -NH-CO-NH- ;

      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français numéros 2 320 330, 2 270 846, 2 316 271, 2 336 434 et 2 413 907 et les brevets US numéros 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4.026.945 et 4 027 020.
      On peut utiliser plus particulièrement les polymères qui sont constitués de motifs récurrents répondant à la formule : 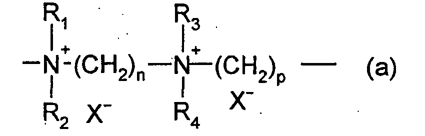 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (a) particulièrement préféré est celui pour lequel R₁, R₂, R₃ et R₄ représentent un groupe méthyle et n = 3, p= 6 et X = Cl, dénommé "Hexadimethrine chloride" selon la nomenclature INCI (CTFA).
(8) les polycondensats de polyammonium quaternaires constitués de motifs de formule (VIII): 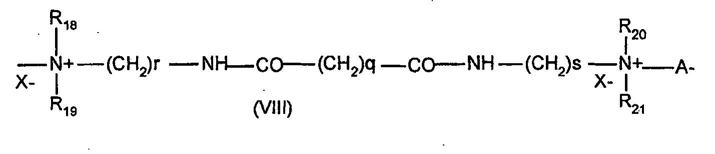 formule dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH,
      où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
      A désigne un groupe d'un dihalogénure ou représente de préférence -CH₂-CH₂-O-CH₂-CH₂-
      De tels composés sont notamment décrits dans la demande de brevet EP-A-122 324.
      On peut, par exemple, citer parmi ceux-ci, les produits "Mirapol^{®} A 15", "Mirapol^{®} AD1", "Mirapol^{®} AZ1" et "Mirapol^{®} 175" vendus par la société Miranol.
(9) les homopolymères ou copolymères dérivés des acides acrylique ou méthacrylique et comportant des motifs : 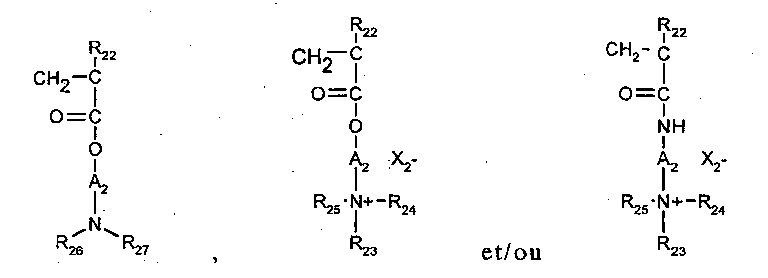 dans lesquels les groupements R₂₂ désignent indépendamment H ou CH₃,
   les groupements A₂ désignent indépendamment un groupe alkyle linéaire ou ramifié de 1 à 6 atomes de carbone, ou un groupe hydroxyalkyle de 1 à 4 atomes de carbone,
   les groupements R₂₃, R₂₄, R₂₅, identiques ou différents, désignant indépendamment un groupe alkyle de 1 à 18 atomes de carbone, ou un groupe benzyle,
   les groupements R₂₆ et R₂₇ représentent un atome d'hydrogène ou un groupement alkyle de 1 à 6 atomes de carbone,
   X₂⁻ désigne un anion, par exemple méthosulfate ou halogénure tel que chlorure ou bromure.
   Le ou les comonomères utilisables dans la préparation des copolymères correspondants appartiennent à la famille des acrylamides, méthacrylamides, diacétone-acrylamides, acrylamides et méthacrylamides substitués à l'azote par des alkyle inférieurs, des esters d'alkyle, des acides acrylique ou méthacrylique, la vinylpyrrolidone ou des esters vinyliques.
(10) Les polymères quaternaires de vinylpyrrolidone et de vinylimidazole.
(11) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bis-acrylamide.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les homopolymères et copolymères d'halogénure de dialkyldiallylammonium, les polyéthylène-imines, les polycondensats à motifs récurrents diammonium ou polyammonium quaternaire.

Comme autres polymères cationiques convenant dans la présente invention, on peut notamment citer les polymères cellulosiques, par exemple, les dérivés d'éthers de cellulose comportant des groupements ammonium quaternaires décrits dans le brevet français N° 1 492 597.

On peut également citer les copolymères de cellulose ou les dérivés de cellulose greffés avec un monomère hydrosoluble d'ammonium quaternaire, et décrits notamment dans le brevet US numéro 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.

On peut également citer les gommes de guar.

Les polymères cationiques décrits ci-dessus sont présents de préférence en une quantité allant de 0,01 à 10 % en poids, mieux encore de 0,05 à 5 % en poids, préférentiellement de 0,1 à 3 % en poids, et encore plus préférentiellement de 0,5 à 4,5 % en poids par rapport au poids total de la composition.

Les sels hydrosolubles minéraux ou organiques, pouvant être utilisés dans la présente invention, sont choisis parmi les sels hydrosolubles de métaux monovalents ou divalents, par exemple de métaux alcalins ou alcalino-terreux, ou d'ammonium ou d'amines, et des acides minéraux ou des acides organiques carboxyliques dont l'anion comportent de 1 à 7 atomes de carbone. Ils présentent de préférence une masse molaire comprise entre 25 et 650 g/mol.

A titre d'exemples de tels sels, on peut notamment citer le chlorure de sodium, le chlorure de potassium, le chlorure de calcium, le chlorure de magnésium, le chlorure d'ammonium, le chlorure de monoéthanolamine, le citrate de sodium, le citrate d'ammonium, le sulfate de magnésium et les sels de sodium de l'acide phosphorique. De préférence, on utilise les sels de métaux monovalents et le chlorure de sodium est particulièrement préféré.

Les sels hydrosolubles sont présents en une quantité supérieure à 1% en poids, de préférence en une quantité allant de 1 à 25% en poids, mieux encore de 3 à 10% en poids, et encore plus préférentiellement de 4 à 8 % en poids par rapport au poids total de la composition.

Le milieu aqueux cosmétiquement acceptable est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement compris entre 2 et 11, et de préférence entre 3 et 10.

La composition selon l'invention peut comprendre en outre un ou plusieurs additifs classiques bien connus dans la technique, tels que des épaississants polymériques naturels ou synthétiques, anioniques, amphotères, zwittérioniques, non ioniques ou cationiques, associatifs ou non, des épaississants non polymériques comme des acides ou des électrolytes, des agents tensioactifs cationiques, des agents nacrants, des agents opacifiants, des colorants ou pigments, des parfums, des huiles minérales, végétales et/ou synthétiques, des cires dont les céramides, des vitamines, des filtres UV, des agents anti-radicalaires, des agents plastifiants, des agents conservateurs ou des agents de stabilisation du pH.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions conformes à l'invention peuvent être utilisées pour le lavage, le lavage et le conditionnement des matières kératiniques, en particulier des cheveux, par exemple, comme shampoings conditionneurs.

Un autre objet de l'invention est un procédé de traitement cosmétique des matières kératiniques, telles que les cheveux, qui consiste à appliquer une quantité efficace d'une composition telle que décrite ci-dessus, sur lesdites matières, à rincer après un éventuel temps de pose.

Les exemples suivants illustrent la présente invention. Les quantités indiquées ci-après sont exprimées en pourcentage en poids par rapport au poids total de la composition, sauf indication contraire.

### EXEMPLES

### Préparation de compositions selon l'invention

Les compositions 1 à 3, selon l'invention, ont été préparées à partir des ingrédients indiqués dans le tableau ci-dessous.

Toutes les concentrations ci-dessous sont indiquées en % en poids de matières actives.

| Composition | 1 | 2 | 3 |
|---|---|---|---|
| Lauryléthersulfate de sodium (2,2 moles d'oxyde d'éthylène)⁽¹⁾ | 5 % | 5 % | 5 % |
| Disodium cocamphodiacétate ⁽²⁾ | 5 % | - | - |
| Cocoylamidopropylbétaïne ⁽³⁾ | - | 10 | 10 % |
| Poly(chlorure de diméthyldiallylammonium)⁽⁴⁾ Densité de charge = 6,2 mEq/g | 0,5 % | 0,5 % | - |
| Polyéthylène imine⁽⁵⁾ Densité de charge = 16 mEq/g | - | - | 4 % |
| NaCl total | 4,7 % | 5,7 % | 5,7 % |
| Eau qsp | 100 % | 100 % | 100 % |
| pH ajusté à | 7 | 7 | 7 |

| | | | |
|---|---|---|---|
| ⁽¹⁾ vendu sous la dénomination commerciale Texapon^{®} N 702 par la société COGNIS. ⁽²⁾ vendu sous la dénomination commerciale Miranol^{®} C2M par la société RHODIA CHIMIE. ⁽³⁾ vendu sous la dénomination commerciale TegoBetaine^{®} F50 par la société Goldschmidt AG. ⁽⁴⁾ vendu sous la dénomination commerciale Merquat^{®} 100 par la société ONDEO-NALCO. ⁽⁵⁾ vendu sous la dénomination commerciale Lupasol^{®} G-35 par la société BASF. | | | |

Appliquées sur des cheveux naturels en tant que shampooings, ces compositions leur confèrent un haut niveau de démêlage tant sur cheveux mouillés que sur cheveux secs.

## Revendications

1. Composition cosmétique détergente comprenant, dans un milieu aqueux cosmétiquement acceptable,
- au moins un tensioactif anionique et au moins un tensioactif amphotère choisi parmi les alkyl(C₈-C₂₄)amidoalkyl(C₃-C₈)-bétaïnes, les sulfobétaïnes, les alkyl(C₈-C₂₄)amidoalkyl(C₆-C₈)sulfobétaïnes, les alkyl(C₈-C₂₄)amphomonoacétates, les alkyl(C₈-C₂₄)amphodiacétates, les alkyl(C₈-C₂₄)amphomonopropionates, les alkyl(C₈-C₂₄)amphodipropionates et les phosphobétaïnes, en un rapport pondéral tensioactif(s) anionique(s)/tensioactif(s) amphotère(s) est inférieur ou égal à 1,
- au moins un polymère cationique présentant une densité de charge cationique supérieure à 5 méq/g,
- au moins 1 % en poids, par rapport au poids total de la composition, d'au moins un sel hydrosoluble minéral ou organique, l'anion de ce dernier comportant de 1 à 7 atomes de carbone,
la quantité totale de tensioactifs dans la composition étant inférieure ou égale à 18 % en poids par rapport au poids total de la composition.

2. Composition détergente selon la revendication 1, **caractérisée en ce que** le rapport pondéral tensioactif(s) anionique(s)/tensioactif(s) amphotère(s) est compris entre 0,1 et 1, de préférence entre 0,2 et 1.

3. Composition selon la revendication 1 ou 2, **caractérisée en ce que** la quantité totale de tensioactifs dans la composition est comprise entre 4 et 18 %, de préférence entre 6 et 15% en poids, par rapport au poids total de la composition.

4. Composition selon l'une quelconque des revendications précédentes, **caractérisée en ce que** le tensioactif anionique est choisi parmi les sels de métaux alcalins, les sels d'ammonium, les sels d'amines, les sels d'aminoalcools ou les sels de métaux alcalino-terreux, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 8 à 22 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle de tous ces composés comportant de 12 à 20 atomes de carbone.

5. Composition selon la revendication 4, **caractérisée en ce que** le tensioactif anionique est choisi parmi les sulfates d'alkyle, les alkyléther-sulfates, de préférence à 2 ou 3 moles d'oxyde d'éthylène, et les alkyléthercarboxylates, les groupes alkyle comportant de 6 à 24 atomes de carbone, sous la forme de sels de sodium, de magnésium ou d'ammonnium.

6. Composition selon l'une quelconque des revendications précédentes, **caractérisée en ce que** le tensioactif amphotère est choisi parmi les alkyl(C₁₀-C₂₄)amidoalkyl(C₃-C₈)-bétaïnes et les alkyl(C₁₀-C₂₄)amphodiacétates.

7. Composition selon l'une quelconque des revendications précédentes, **caractérisée en ce que** le polymère cationique de charge supérieure à 5 méq/g est soluble dans ladite composition.

8. Composition selon la revendication 7, **caractérisée en ce que** la composition est transparente.

9. Composition cosmétique transparente selon l'une quelconque des revendications précédentes, **caractérisée en ce que** le polymère cationique présente une densité de charge comprise entre 5 et 20 méq/g.

10. Composition selon l'une quelconque des revendications précédentes, **caractérisée en ce que** le polymère cationique est choisi parmi les homopolymères et copolymères d'halogénure de dialkyldiallylammonium, les polyéthylène-imines, les polycondensats à motifs récurrents diammonium ou polyammonium quaternaire.

11. Composition selon l'une quelconque des revendications précédentes, **caractérisée en ce qu'**elle comprend le polymère cationique en une quantité comprise entre 0,01 et 10% en poids, de préférence entre 0,05 et 5% en poids par rapport au poids total de la composition.

12. Composition selon l'une quelconque des revendications précédentes, **caractérisée en ce que** le sel hydrosoluble présente une masse molaire comprise entre 25 et 650 g/mole.

13. Composition selon l'une quelconque des revendications précédentes, **caractérisée en ce que** le sel hydrosoluble est choisi parmi le chlorure de sodium, le chlorure de potassium, le chlorure de calcium, le chlorure de magnésium, le chlorure d'ammonium, le chlorure de monoéthanolamine, le citrate de sodium, le citrate d'ammonium, le sulfate de magnésium et les sels de sodium de l'acide phosphorique.

14. Composition selon l'une quelconque des revendications précédentes, **caractérisée en ce qu'**elle comprend le ou les sels hydrosolubles en une quantité allant de 1 à 25% en poids, de préférence de 3 à 10% en poids par rapport au poids total de la composition.

15. Composition selon l'une quelconque des revendications précédentes, **caractérisée en ce que** le milieu aqueux cosmétiquement acceptable est constitué d'eau ou d'un mélange d'eau et d'au moins un solvant organique.

16. Composition selon l'une quelconque des revendications précédentes, **caractérisée en ce qu'**elle comprend en outre au moins un additif choisi parmi des épaississants polymériques naturels ou synthétiques, anioniques, amphotères, zwittérioniques, non ioniques ou cationiques, associatifs ou non, des épaississants non polymériques, des agents tensioactifs cationiques, des agents nacrants, des agents opacifiants, des colorants ou pigments, des parfums, des huiles minérales, végétales et/ou synthétiques, des cires, des vitamines, des filtres UV, des agents anti-radicalaires, des agents plastifiants, des agents conservateurs ou des agents de stabilisation du pH.

17. Utilisation de la composition selon l'une quelconque des revendications 1 à 16, pour le lavage des matières kératiniques.

18. Utilisation de la composition selon l'une quelconque des 1 à 16, pour le lavage et le conditionnement des matières kératiniques.

19. Procédé de traitement cosmétique des matières kératiniques, comprenant l'application d'une quantité efficace d'une composition cosmétique selon l'une quelconque des revendications 1 à 16, sur lesdites matières, à rincer après un éventuel temps de pose.

## Claims

1. Detergent cosmetic composition comprising, in a cosmetically acceptable aqueous medium:
- at least one anionic surfactant and at least one amphoteric surfactant chosen from (C8-C24)alkylamido(C3-C8)alkylbetaines, sulfobetaines, (C8-C24)alkylamido(C6-C8)alkylsulfobetaines, (C8-C24)alkyl amphomonoacetates, (C8-C24)alkyl amphodiacetates, (C8-C24)alkyl amphomonopropionates, (C8-C24)alkyl amphodipropionates and phosphobetaines, in an anionic surfactant(s)/amphoteric surfactant(s) weight ratio of less than or equal to 1,
- at least one cationic polymer with a cationic charge density of greater than 5 meq/g,
- at least 1% by weight, relative to the total weight of the composition, of at least one mineral or organic water-soluble salt, the anion of this salt comprising from 1 to 7 carbon atoms,
the total amount of surfactants in the composition being less than or equal to 18% by weight relative to the total weight of the composition.

2. Detergent composition according to Claim 1, **characterized in that** the anionic surfactant(s)/amphoteric surfactant(s) weight ratio is between 0.1 and 1 and preferably between 0.2 and 1.

3. Composition according to Claim 1 or 2, **characterized in that** the total amount of surfactants in the composition is between 4% and 18% and preferably between 6% and 15% by weight relative to the total weight of the composition.

4. Composition according to any one of the preceding claims, **characterized in that** the anionic surfactant is chosen from alkali metal salts, ammonium salts, amine salts, amino alcohol salts or alkaline-earth metal salts, of the following compounds: alkyl sulfates, alkyl ether sulfates, alkylamido ether sulfates, alkylarylpolyether sulfates, monoglyceride sulfates; alkylsulfonates, alkyl phosphates, alkylamidesulfonates, alkylarylsulfonates, α-olefin sulfonates, paraffin sulfonates; alkylsulfosuccinates, alkyl ether sulfosuccinates, alkylamide sulfosuccinates; alkylsulfoacetates; acylsarcosinates; and acylglutamates, the alkyl and acyl groups of all these compounds containing from 8 to 22 carbon atoms and the aryl group preferably denoting a phenyl or benzyl group; C₆-C₂₄ alkyl esters of polyglycoside carboxylic acids; alkylsulfosuccinamates, acylisethionates and N-acyltaurates, the alkyl or acyl group of all these compounds containing from 12 to 20 carbon atoms.

5. Composition according to Claim 4, **characterized in that** the anionic surfactant is chosen from alkyl sulfates, alkyl ether sulfates, preferably containing 2 or 3 mol of ethylene oxide, alkyl ether carboxylates, the alkyl groups containing from 6 to 24 carbon atoms, in the form of sodium, magnesium or ammonium salts.

6. Composition according to any one of the preceding claims, **characterized in that** the amphoteric surfactant is chosen from (C₁₀-C₂₄) alkylamido (C₃-C₈) alkylbetaines and (C₁₀-C₂₄) alkyl amphodiacetates.

7. Composition according to any one of the preceding claims, **characterized in that** the cationic polymer with a charge of greater than 5 meq/g is soluble in the said composition.

8. Composition according to Claim 7, **characterized in that** the composition is transparent.

9. Transparent cosmetic composition according to any one of the preceding claims, **characterized in that** the cationic polymer has a charge density of between 5 and 20 meq/g.

10. Composition according to any one of the preceding claims, **characterized in that** the cationic polymer is chosen from dialkyldiallylammonium halide homopolymers and copolymers, polyethyleneimines and polycondensates containing diquaternary ammonium or polyquaternary ammonium repeating units.

11. Composition according to any one of the preceding claims, **characterized in that** it comprises the cationic polymer in an amount of between 0.01% and 10% by weight and preferably between 0.05% and 15% by weight relative to the total weight of the composition.

12. Composition according to any one of the preceding claims, **characterized in that** the water-soluble salt has a molar mass of between 25 and 650 g/mol.

13. Composition according to any one of the preceding claims, **characterized in that** the water-soluble salt is chosen from sodium chloride, potassium chloride, calcium chloride, magnesium chloride, ammonium chloride, monoethanolamine chloride, sodium citrate, ammonium citrate, magnesium sulfate and the sodium salts of phosphoric acid.

14. Composition according to any one of the preceding claims, **characterized in that** it comprises the water-soluble salt(s) in an amount ranging from 1% to 25% by weight and preferably from 3% to 10% by weight relative to the total weight of the composition.

15. Composition according to any one of the preceding claims, **characterized in that** the cosmetically acceptable aqueous medium consists of water or of a mixture of water and of at least one organic solvent.

16. Composition according to any one of the preceding claims, **characterized in that** it also comprises at least one additive chosen from natural or synthetic, anionic, amphoteric, zwitterionic, nonionic or cationic, associative or non-associative polymeric thickeners, non-polymeric thickeners, cationic surfactants, nacreous agents, opacifiers, dyes or pigments, fragrances, mineral, plant and/or synthetic oils, waxes, vitamins, UV-screening agents, free-radical scavengers, plasticizers, preserving agents or pH stabilizers.

17. Use of the composition according to any one of Claims 1 to 16, for washing keratin materials.

18. Use of the composition according to any one of Claims 1 to 16, for washing and conditioning keratin materials.

19. Cosmetic process for treating keratin materials, comprising the application of an effective amount of a cosmetic composition according to any one of Claims 1 to 16 to the said materials, and rinsing after an optional leave-in time.

## Patentansprüche

1. Reinigende kosmetische Zusammensetzung, die in einem kosmetisch akzeptablen, wässrigen Medium enthält:
- mindestens einen anionischen grenzflächenaktiven Stoff und mindestens einen amphoteren grenzflächenaktiven Stoff, der unter den Alkyl(C₈₋₂₄)amidoalkyl(C₃₋₈)betainen, Sulfobetainen, Alkyl(C₈₋₂₄)amidoalkyl(C₆₋₈)sulfobetainen, Alkyl(C₈₋₂₄)amphomonoacetaten, Alkyl(C₈₋₂₄)amphodiacetaten, Alkyl(C₈₋₂₄)amphomonopropionaten, Alkyl(C₈₋₂₄)amphodipropionaten und Phosphobetainen ausgewählt ist, in einem Gewichtsverhältnis anionische(r) grenzflächenaktive(r) Stoff(e) / amphotere(r) grenzflächenaktive(r) Stoff(e) von 1 oder darunter,
- mindestens ein kationisches Polymer, das eine kationische Ladungsdichte über 5 meq/g aufweist,
- mindestens 1 Gew.-%, bezogen auf das Gesamtgewicht der Zusammensetzung, mindestens eines anorganischen oder organischen, wasserlöslichen Salzes, wobei das Anion des organischen Salzes 1 bis 7 Kohlenstoffatome aufweist,
wobei die Gesamtmenge der grenzflächenaktiven Stoffe in der Zusammensetzung höchstens 18 Gew.-% beträgt, bezogen auf das Gesamtgewicht der Zusammensetzung.

2. Reinigende Zusammensetzung nach Anspruch 1, **dadurch gekennzeichnet, dass** das Gewichtsverhältnis anionische(r) grenzflächenaktive(r) Stoff(e)/amphotere(r) grenzflächenaktive(r) Stoff(e) im Bereich von 0,1 bis 1 und vorzugsweise 0,2 bis 1 liegt.

3. Zusammensetzung nach Anspruch 1 oder 2, **dadurch gekennzeichnet, dass** die Gesamtmenge der grenzflächenaktiven Stoffe in der Zusammensetzung im Bereich von 4 bis 18 Gew.-% und vorzugsweise 6 bis 15 Gew.-%, bezogen auf das Gesamtgewicht der Zusammensetzung liegt.

4. Zusammensetzung nach einem der vorhergehenden Ansprüche, **dadurch gekennzeichnet, dass** der anionische grenzflächenaktive Stoff ausgewählt ist unter den Alkalimetallsalzen, Ammoniumsalzen, Aminsalzen, Aminoalkoholsalzen oder Erdalkalisalzen der folgenden Verbindungen: Alkylsulfate, Alkylethersulfate, Alkylamidoethersulfate, Alkylarylpolyethersulfate, Monoglyceridsulfate; Alkylsulfonate, Alkylphosphate, Alkylamidsulfonate, Alkylarylsulfonate, α-Olefinsulfonate, Paraffinsulfonate; Alkylsulfosuccinate, Alkylethersulfosuccinate, Alkylamidsulfosuccinate; Alkylsulfoacetate; Acylsarcosinate; und Acylglutamate, wobei die Alkyl- oder Acylgruppen aller dieser Verbindungen 8 bis 22 Kohlenstoffatome aufweisen und die Arylgruppe vorzugsweise eine Phenylgruppe oder Benzylgruppe bedeutet; C₆₋₂₄-Alkylestern von Polyglycosidcarbonsäuren; Alkylsulfosuccinamaten, Acylisethionaten und N-Acyltauraten, wobei die Alkyl- oder Acylgruppe aller dieser Verbindungen 12 bis 20 Kohlenstoffatome enthält.

5. Zusammensetzung nach Anspruch 4, **dadurch gekennzeichnet, dass** der anionische grenzflächenaktive Stoff unter den Alkylsulfaten, Alkylethersulfaten mit vorzugsweise 2 oder 3 mol Ethylenoxid und Alkylethercarboxylaten , wobei die Alkylgruppe 6 bis 24 Kohlenstoffatome enthält, in der Form der Natriumsalze, Magnesiumsalze oder Ammoniumsalze ausgewählt ist.

6. Zusammensetzung nach einem der vorhergehenden Ansprüche, **dadurch gekennzeichnet, dass** der amphotere grenzflächenaktive Stoff unter den Alkyl(C₁₀₋₂₄)amidoalkyl(C₃₋₈)betainen und Alkyl(C₁₀₋₂₄)amphodiacetaten ausgewählt ist.

7. Zusammensetzung nach einem der vorhergehenden Ansprüche, **dadurch gekennzeichnet, dass** das kationische Polymer mit einer Ladungsdichte über 5 meq/g in der Zusammensetzung löslich ist.

8. Zusammensetzung nach Anspruch 7, **dadurch gekennzeichnet, dass** die Zusammensetzung transparent ist.

9. Transparente kosmetische Zusammensetzung nach einem der vorhergehenden Ansprüche, **dadurch gekennzeichnet, dass** das kationische Polymer eine Ladungsdichte im Bereich von 5 bis 20 meq/g aufweist.

10. Zusammensetzung nach einem der vorhergehenden Ansprüche, **dadurch gekennzeichnet, dass** das kationische Polymer unter den Homopolymeren und Copolymeren von Dialkylallylammoniumhalogeniden, Polyethyleniminen, Polykondensaten mit wiederkehrenden quartären Diammonium- oder Polyammoniumeinheiten ausgewählt ist.

11. Zusammensetzung nach einem der vorhergehenden Ansprüche, **dadurch gekennzeichnet, dass** sie das kationische Polymer in einer Menge von 0,01 bis 10 Gew.-% und vorzugsweise 0,05 bis 5 Gew.-%, bezogen auf das Gesamtgewicht der Zusammensetzung, enthält.

12. Zusammensetzung nach einem der vorhergehenden Ansprüche, **dadurch gekennzeichnet, dass** das wasserlösliche Salz eine Molmasse von 25 bis 650 g/mol aufweist.

13. Zusammensetzung nach einem der vorhergehenden Ansprüche, **dadurch gekennzeichnet, dass** das wasserlösliche Salz unter Natriumchlorid, Kaliumchlorid, Calciumchlorid, Magnesiumchlorid, Ammoniumchlorid, Monoethanolaminchlorid, Natriumcitrat, Ammoniumcitrat, Magnesiumsulfat und den Natriumsalzen von Phosphorsäure ausgewählt ist.

14. Zusammensetzung nach einem der vorhergehenden Ansprüche, **dadurch gekennzeichnet, dass** sie das oder die wasserlösliche(n) Salz(e) in einer Menge von 1 bis 25 Gew.-% und vorzugsweise 3 bis 30 Gew.-%, bezogen auf das Gesamtgewicht der Zusammensetzung, enthält.

15. Zusammensetzung nach einem der vorhergehenden Ansprüche, **dadurch gekennzeichnet, dass** das kosmetisch akzeptable, wässrige Medium aus Wasser oder einem Gemisch von Wasser und mindestens einem organischen Lösungsmittel besteht.

16. Zusammensetzung nach einem der vorhergehenden Ansprüche, **dadurch gekennzeichnet, dass** sie ferner mindestens einen Zusatzstoff enthält, der unter den natürlichen oder synthetischen, anionischen, amphoteren, zwitterionischen, nichtionischen oder kationischen, assoziativen oder nichtassoziativen polymeren Verdickungsmitteln, nichtpolymeren Verdickungsmitteln, kationischen grenzflächenaktiven Stoffen, Perlglanzstoffen, Trübungsmitteln, Farbstoffen oder Pigmenten, Parfums, Mineralölen, pflanzlichen Ölen und/oder synthetischen Ölen, Wachsen, Vitaminen, UV-Filtern, Radikalfängern für freie Radikale, Weichmachern, Konservierungsmitteln oder pH-Stabilisatoren ausgewählt sind.

17. Verwendung der Zusammensetzung nach einem der Ansprüche 1 bis 16 für die Reinigung von Keratinsubstanzen.

18. Verwendung der Zusammensetzung nach einem der Ansprüche 1 bis 16 für die Reinigung und Konditionierung von Keratinsubstanzen.

19. Verfahren zur kosmetischen Behandlung von Keratinsubstanzen, das das Aufbringen einer kosmetischen Zusammensetzung nach einem der Ansprüche 1 bis 16 in einer wirksamen Menge auf die Keratinsubstanzen und Spülen nach einer gegebenenfalls abgewarteten Einwirkzeit umfasst.
